# EUROPÄISCHE PATENTSCHRIFT

(11) **EP 0 783 656 B1**
(45) Veröffentlichungstag und Bekanntmachung des Hinweises auf die Patenterteilung: **20.01.1999**
(21) Anmeldenummer: 95936493.6
(22) Anmeldetag: 14.10.1995
(51) Int. Cl.: F24F 3/12, F16H 31/00

(54) **DUFTVERDUNSTER, INSBESONDERE FÜR TOILETTEN**
FRAGRANCE EVAPORATOR, IN PARTICULAR FOR TOILETS
EVAPORATEUR A PARFUMS, NOTAMMENT POUR TOILETTES

(30) Priorität: 17.10.1994 DE 4437019; 05.11.1994 DE 4439555; 18.11.1994 DE 4441105
(43) Veröffentlichungstag der Anmeldung: 16.07.1997
(60) Teilanmeldung: 97121512.4
(73) Patentinhaber: VENTA VERTRIEBS AG, 6331 Hünenberg (CH)
(72) Erfinder: HITZLER, Alfred, D-88284 Mochenwangen (DE); HARTER, Erich, D-88284 Mochenwangen (DE)
(74) Vertreter: Patentanwälte Eisele, Otten & Roth
(86) Internationale Anmeldenummer: EP9504042
(87) Internationale Veröffentlichungsnummer: WO9612143

(56) Entgegenhaltungen:
- WO-A-90/02231
- DE-A- 2 424 140
- DE-C- 3 412 745
- FR-A- 1 191 751
- FR-A- 2 097 434
- FR-A- 2 595 839
- US-A- 3 465 605
- US-A- 4 261 930

## Beschreibung

Die Erfindung betrifft einen Duftverdunster, insbesondere für Toiletten nach dem Oberbegriff des Anspruchs 1.

Um unangenehme Gerüche, beispielsweise in Toilettenanlagen von Gaststätten, sonstigen öffentlichen Gebäuden, aber auch in Gebäuden für den Privatgebrauch, zu vermeiden oder zu unterdrücken wurden bislang häufig Versprühmittel eingesetzt. Diese vernebeln einen flüssigen Duftstoff in feinen Tröpfchen in der Raumluft. Ein Teil dieser Tröpfchen verdunstet nahezu vollständig und befindet sich somit in der Raumluft, die dadurch den Duft dieses Duftstoffes aufweist.

Allerdings geschieht in der Regel die Verdunstung des versprühten Duftstoffes nicht vollständig. Ein Teil der vernebelten Tröpfchen setzt sich auf den Boden, den Wänden, auf Einrichtungsgegenständen und auf der Kleidung von im Raum befindlichen Personen ab. Dies führt zu unangenehmen Nebeneffekten. So wird der Boden unter Umständen glitschig oder Kleidung und Schuhe werden von den zum Teil aggressiven Duftstoffen, beispielsweise von ätherischen Ölen, angegriffen. In öffentlichen Toilettenanlagen muß aufgrund dessen der Boden regelmäßig in Intervallen von ca. 2 Std. gewischt werden.

Desweiteren sind zur Vermeidung unangenehmer Gerüche sogenannte Geruchssteine bekanntgeworden. Derartige Geruchssteine weisen den Nachteil auf, daß sie ständig ihre Duftnote an die umgebende Raumluft abgeben. Dies führt zu einer Verschwendung von Duftstoff, da dieser auch in die Raumluft abgegeben wird, wenn kein Fremdgeruch bekämpft werden muß, beispielsweise wenn die Toilette nicht benutzt wird. Dieser Mangel läßt sich bei Geruchssteinen auch nicht ohne weiteres beheben, da die Duftstoffabgabe bei Geruchssteinen nicht regulierbar ist.

Aus der DE-34 12 745 ist ein Luftbefeuchtungs/Luftreinigungsgerät bekannt geworden, welches nach dem sogenannten Kaltverdunstungsprinzip arbeitet. Dabei ist ein, in einer Flüssigkeit rotierender Plattenstapel vorgesehen, wobei dem üblicherweise verwendeten Wasser als Flüssigkeit eventuell gewisse Duftstoffe tropfenweise zuführbar sind. Derartige Geräte dienen zur Luftbefeuchtung bei trockener Heizungsluft bzw. zur Luftreinigung und zur Absorption von Schadstoffteilen an den benetzten Plattenoberflächen.

Aufgabe der Erfindung ist es daher, einen Duftverdunster vorzuschlagen, bei dem der Duftstoff regulierbar nur bei Bedarf und in dosierbarer Menge der Raumluft zuführbar ist.

Diese Aufgabe wird ausgehend von einem Duftverdunster der einleitend genannten Art durch die kennzeichnenden Merkmale des Anspruchs 1 gelöst.

Durch die in den Unteransprüchen genannten Maßnahmen sind vorteilhafte Ausführungen und Weiterbildungen der Erfindung möglich.

Demgemäß weist ein erfindungsgemäßer Duftverdunster ein Duftstoffreservoir auf das als separate, dem Gehäuse (2) des Duftverdunsters (1) entnehmbare Duftstoffwanne (15) ausgebildet ist, in welches der Plattenstapel eingesetzt ist, wobei der Antrieb für den Plattenstapel über einen Hebelantrieb (7, 6) erfolgt.

Durch die Verteilung des flüssigen Duftstoffes auf der festen Oberfläche findet eine erhöhte Verdunstung statt. Da der die feste Oberfläche umgebende Verdunstungsraum mit der Raumluft in Verbindung steht, kann somit der gewünschte Dufteffekt durch das Einbringen von mit Duftstoff angereicherter Luft aus dem Verdunstungsraum des Duftverdunsters in den geruchsbelasteten Raum bewirkt werden. Die feste Oberfläche wird nur bei Bedarf mit flüssigem Duftstoff benetzt, so daß kein unnötiger Duftstoff in die Raumluft verteilt wird. Boden, Wände, Einrichtungsgegenstände oder auch Kleidung einer im Raum befindlichen Person werden nicht von Duftstoff benetzt. Da ein Duftstoffreservoir vorgesehen ist, steht auch über einen längeren Zeitraum eine ausreichende Menge von Duftstoff zur Verfügung.

Vorteilhafterweise wird die Oberfläche so beweglich angeordnet, daß sie wenigstens teilweise zum Benetzen mit Duftstoff in ein Duftstoffbad eintauchbar ist und die durch das Eintauchen benetzte Oberfläche nach dem Eintauchen aus dem Duftstoffbad in den Verdunstungsraum einfahrbar ist.

Auf diese Weise wird zumindest der eingetauchte Teil der Oberfläche vollständig mit dem Duftstoff benetzt. Ein etwaiger verbliebener Rest von Duftstoff auf der Oberfläche vor dem Eintauchen schadet hierbei nicht, sondern wird beim Eintauchen dem Duftstoffreservoir wieder zugeführt. Nach dem Einfahren der benetzten Oberfläche in den Verdunstungsraum kann dann die Verdunstung des Duftstoffes stattfinden.

Bei der vorliegenden Erfindung wird weiterhin ein Plattenstapel vorgesehen, bei dem zwischen den einzelnen Platten ein Spalt offen steht, wobei die Oberfläche der Platten die zu benetzende Oberfläche für den flüssigen Duftstoff bildet. Durch eine solche lamellenartige Anordnung von Platten in einem Plattenstapel ergibt sich eine sehr große benetzte Oberfläche beim Eintauchen des Plattenstapels in das Duftstoffbad bei vergleichsweise kleinem Außenmaß. Durch diese große Oberfläche kommt es zu einer vermehrten Verdunstung von Duftstoff, sobald der benetzte Teil des Plattenstapels in den Verdunstungsraum eingebracht wird.

In einer bevorzugten Ausführungsform wird der Plattenstapel zylinderförmig und rotierbar ausgebildet. Ein derart gestalteter Plattenstapel liegt mit seiner Rotationsachse parallel zur Flüssigkeitsoberfläche des Duftstoffbades, so daß ständig ein Teil der Oberfläche des Plattenstapels sich in dem Duftstoffbad befindet, während ständig ein anderer, benetzter Teil der Oberfläche des Plattenstapels den Verdunstungsraum während der Rotationsbewegung durchquert. Hierdurch wird eine kontinuierliche und vollständige Benetzung der Plattenstapeloberfläche zuverlässig gewährleistet.

Vorzugsweise wird ein Flügelrad als Ventilator zur Belüftung des Verdunstungsraums vorgesehen. Durch den Ventilator und die dadurch verursachte Abfuhr der mit Duftstoff angereicherten Luft aus dem Duftverdunster und die ständige Strömung von Frischluft über die benetzte Oberfläche im Verdunstungsraum wird eine Verstärkung des Verdunstungsvorgangs erzielt. Somit kann der Verdunstungsraum und seine entsprechenden Öffnungen mit relativ kompakter Größe ausgeführt werden, wodurch die Gesamtbaugröße des Duftverdunsters positiv beeinflußt wird.

Empfehlenswert ist es, für den Antrieb des Plattenstapels und des Ventilators denselben Motor vorzusehen, da hierdurch ein zweiter Antriebsmotor für den Plattenstapel bzw. für den Ventilator eingespart werden kann.

Vorteilhafterweise wird ein erfindungsgemäßer Duftverdunster nicht im Dauerbetrieb verwendet. Es empfiehlt sich vielmehr, eine Steuerung für den Duftverdunster vorzusehen, die einen intermittierenden Betrieb, d. h. eine Betriebsart mit zwischenzeitlichem Stillstand, erlaubt. Hierdurch kommt der Spareffekt, der gegenüber einem Duftverdunster mit kontinuierlicher Verdunstung hinsichtlich der benötigten Duftstoffmenge möglich ist, voll zum Tragen. Zudem wird hierdurch auch der Stromverbrauch abgesenkt, so daß bei einem Batterie- oder Akkubetrieb die Stromversorgung über einen längeren Zeitraum ohne Batterietausch oder Aufladen eines Akkumulators erhalten bleibt.

In einer vorteilhaften Ausführung der Erfindung wird eine Steuerung vorgesehen, bei der die Betriebszeiten des Verdunsters durch Sensoren verschiedenster Art und/oder durch ein oder mehrere Zeitintervalle steuerbar ist. Als Sensor kommen hierbei der Türschalter der Eingangstür des Raumes, der Lichtschalter des Raumes oder ein unabhängig davon arbeitender Lichtsensor, ein Sensor, der auf die Wasserspülung reagiert, beispielsweise ein Druckschalter in einer Toilette, oder ein Sensor, der die Papierentnahme von Toilettenpapier registriert, in Frage. Insbesondere ist der Einsatz eines Geruchssensors von großem Vorteil. Hierdurch ist die Betriebszeit des Duftverdunsters im Hinblick auf ein gutes Ergebnis bei der Aufbereitung der Raumluft optimierbar. Auch andere Sensoren, die unter Umständen aus einfachen Schaltern bestehen können, sind für eine Steuerung eines erfindungsgemäßen Duftverdunsters einsetzbar.

Mit Hilfe von Zeitintervallen sind verschiedene Betriebsarten möglich. So kann mit einer Nachlaufsteuerung, bei der das Gerät per Taste manuell eingeschaltet wird, der Duftverdunster eine bestimmte Zeit, beispielsweise zwischen 30 und 150 Sekunden nach jedem Einschaltvorgang nachlaufen und dann selbständig abschalten.

Je nach Einsatzgebiet kann das Gerät auch auf intermittierenden Betrieb zwischen einem bestimmten Lauf zeitintervall und einer bestimmten Pausenzeit gesteuert werden. Denkbar wäre beispielsweise eine Schaltstellung, in der die Laufzeit 45 Sek. und die Pausenzeit 25 Min. beträgt. In einer solchen Betriebsart wird in regelmäßigen Zeitabständen nach den Pausenzeiten die Raumluft aufgefrischt. Laufzeit und Pausenzeit hängen hierbei von der Größe des Raumes sowie der Intensität der Geruchsbelastung ab.

Die Steuerung kann auch zwischen verschiedenen Betriebsmodi umschaltbar sein. So kann beispielsweise eine Steuerung mit einem Betriebsmodus mit den oben genannten Zeitintervallen von 45 Sek. Laufzeit und 25 Min. Pausenzeit und mit einem weiteren Betriebsmodus von beispielsweise 120 Sek. Laufzeit und 25 Min. Pausenzeit versehen sein. Die Betriebsmodi können für eine bestimmte vorgegebene Zeit ablaufen oder aber auch kontinuierlich automatisch durchlaufen, bis entweder der Betriebsmodus gewechselt oder die Steuerung in eine Schaltstellung gebracht wird, in der der Duftverdunster stillsteht.

Die intermittierende Betriebsweise mit Zeitintervallen kann kombiniert werden mit einer Nachlaufsteuerung, so daß nach manuellem Einschalten des Duftverdunsters sich die Steuerung für ein bestimmtes Zeitintervall in einem intermittierenden Betriebszustand befindet. Sie kann auch mit einem Sensor gekoppelt werden, so daß das Ein- und Ausschalten mit Hilfe der Sensorsignale gesteuert wird und zwischen diesen Zeitpunkten der intermittierende Betriebsmodus läuft.

In der einfachsten Ausführungsform ist der erfindungsgemäße Duftverdunster auch rein manuell betätigbar möglich.

Vorzugsweise wird ein erfindungsgemäßer Duftverdunster mit wenigstens einer Wechselkassette für das Duftstoffbad versehen. So können problemlos verschiedene Duftnoten mit einem Duftverdunster für die Luftaufbereitung eingesetzt werden. Die Duftnoten sind hierdurch einfach und sauber auswechselbar, wodurch ein Duftverdunster gemäß der Erfindung bespielsweise auch für die Verwendung bei einer sogenannten Dufttherapie einsetzbar ist.

Vorzugsweise wird die Wechselkassette für das Duftstoffbad komplett zusammen mit dem Plattenstapel, der mit dem Duftstoff der jeweiligen Duftnote benetzt ist austauschbar ausgebildet und mit einem Deckel versehen. Somit ist ein Vermischen von Duftstoff verschiedener Duftnoten nahezu vollständig ausgeschlossen. Duftstoffe verschiedener Duftnoten kommen durch den Austausch der Wechselkassette nicht miteinander in Berührung. Durch den Abschluß der Wechselkassette mit einer dichtschließenden Haube ist eine solche Wechselkassette ohne Schwierigkeiten transportabel oder kann auch über längere Zeit gelagert werden. Bei einer Lagerung über längere Zeit bestünde ansonsten bei einer offenen Wechselkassette die Gefahr des Austrocknens oder, sofern der Duftstoff in ein flüssiges Trägermedium eingebracht ist, die Gefahr eines "Ausriechens" d. h. des Verlustes des Duftstoffes innerhalb des Trägermediums.

In weiteren Ausführungsformen der Erfindung werden zwei oder mehrere Plattenstapel verwendet. Hierdurch sind nahezu beliebige Außenmaße eines erfindungsgemäßen Duftverdunsters ohne Einbußen bei dessen Effizienz realisierbar.

Beim Antrieb von Walzen, Wellen oder dergleichen, insbesondere auch beim Antrieb eines Plattenstapels eines erfindungsgemäßen Verdunsters, die innerhalb einer Flüssigkeit rotieren, treten immer wieder Probleme wegen Verunreinigungen des Getriebes durch die entsprechende Flüssigkeit auf.

Dies ist insbesondere dann von Nachteil, wenn es sich um aggressive Flüssigkeiten handelt. Bei einem vorliegenden Duftverdunster liegen derartige Flüssigkeiten beispielsweise in Form von ätherischen Ölen vor. Generell stellt sich dieses Problem jedoch beispielsweise auch im Bereich der Chemie, wo häufig mit aggressiven Flüssigkeiten, insbesondere Säuren und Laugen, hantiert wird.

Umfangreiche Abdichtungsmaßnahmen der Getriebeeinheit führen nicht immer zum gewünschten Erfolg. Häufig bleibt als einzige Lösungsmöglichkeit, sämtliche Getriebekomponenten aus Materialien herzustellen, die gegenüber den verwendeten aggressiven Flüssigkeiten resistent sind.

Dies zieht in der Regel einen erhöhten Kostenaufwand nach sich, wobei immer noch das Problem verbleibt, daß die Getriebeeinheit durch die jeweils verwendete Flüssigkeit verschmutzt wird.

Insbesondere beim Wechsel der Flüssigkeit, d. h. bei einem vorliegenden Duftverdunster bei einem Wechsel des Duftstoffbades, kann es zu unerwünschten Vermischungen von Flüssigkeiten kommen. Nicht zuletzt bei chemischen Produktionsanlagen kann es hierbei sogar zu unerwünschten chemischen Reaktionen kommen, sofern das Getriebe nicht äußerst sorgfältig gereinigt wird.

Bei dem erfindungsgemäßen Duftverdunster ist demzufolge ein Antrieb vorgesehen, der wenigstens einen Hebel und ein Zahnrad umfaßt, wobei das Zahnrad drehfest mit dem rotierenden Gegenstand verbunden ist. Außerdem wird eine Vorrichtung zur Lagerung und Führung des Hebels für eine in das Zahnrad eingreifende und mitnehmende Vorwärtsbewegung des Hebels vorgesehen.

Selbst wenn das Zahnrad hierbei durch die Rotationsbewegung in der Flüssigkeit von dieser benetzt wird, besteht der einzige Berührungspunkt mit den weiteren Getriebekomponenten in dem Teil des Hebels, der in das Zahnrad eingreift. Dieser Teil des Hebels ist in der Regel jedoch einfach zu reinigen. Außerdem kann die Bewegung des Hebels so geführt und gesteuert werden, daß für den Fall, daß er tatsächlich mit der Flüssigkeit in Berührung kommt, diese Flüssigkeitsreste in Richtung der Hebelspitze ablaufen und somit in der Tat nur ein äußerstes Ende des Hebels von der Flüssigkeit benetzt wird.

Hierzu wird vorzugsweise der Hebel so orientiert und ausgebildet, daß er mit seiner zum Zahnrad hinweisende Spitze in das Zahnrad eingreift und diese Spitze in jeder Stellung des Hebels nach unten ausgerichtet ist.

Vorteilhafterweise wird die Vorrichtung zur Lagerung und Führung des Hebels so eingerichtet, daß sie eine zwischen zwei Vorwärtsbewegungen des Hebels stattfindende Rückwärtsbewegung erlaubt, bei der sich der Hebel nicht mehr in Eingriff mit dem Zahnrad befindet und dieses nicht berührt.

Somit kann der Hebel das Zahnrad kontinuierlich in einer Drehrichtung antreiben. Zudem kann hierdurch unter anderem auf einen Klinkenantrieb verzichtet werden, der nach Art einer Ratsche in der Rückwärtsbewegung mit seiner in der Vorwärtsbewegung in das Zahnrad eingreifenden Klinke über die Zähne des Zahnrades rutscht. Zum einen entfällt dabei ein für ein Klinkenantrieb zusätzlich erforderliches bewegliches Bauteil, zum anderen wird die in Wohnräumen doch sehr störende Geräuschentwicklung eines Klinkenantriebs vollständig vermieden.

Vorzugsweise umfaßt die Vorrichtung zur Lagerung und Führung des Hebels Gleitflächen, die am Hebel selbst angebracht sind und eine Halterung mit Führungsflächen. Hierdurch kann der Hebel außer einer Schwenkbewegung auch eine Translationsbewegung durchführen, die der Schwenkbewegung überlagert ist. Durch die Überlagerung einer Dreh- und einer Translationsbewegung ist es möglich, die Spitze so anzusteuern, daß sie in Vorwärtsbewegung in das Zahnrad eingreift und in der Rückwärtsbewegung außer Eingriff mit diesem steht.

Vorteilhafterweise wird der Hebel an einem Ende mit einer rotierenden Antriebswelle exzentrisch gelenkig verbunden. Durch eine derartige exzentrisch gelenkige Verbindung mit einer rotierenden Welle wird der Hebel gleichzeitig in eine Schwenk- und Translationsbewegung versetzt, die einander überlagert sind. In Verbindung mit den genannten Gleit- und Führungsflächen kann hierbei nahezu jede beliebige zyklische Bewegung, beispielsweise eine Ellipsenbewegung, der Hebelspitze bewirkt werden. Hierzu müssen die Formen der Gleit- bzw. Führungsflächen sowie die Exzentrizität des Hebelantriebs aufeinander abgestimmt werden.

Vorzugsweise werden bei einem erfindungsgemäßen Antrieb zwei Hebel vorgesehen, die abwechselnd in das Zahnrad eingreifen. Dadurch befindet sich ständig ein Hebel in Eingriff mit dem Zahnrad, so daß dieses nicht nur angetrieben, sonderen auch gegen jede unerwünschte weitere Bewegung, beispielsweise gegen ein Zurückdrehen des rotierenden Gegenstandes nach dem Herausschwenken einer Hebelspitze aus dem Zahnrad, verriegelt ist. Die Rotation des rotierenden Gegenstandes ist somit ständig mechanisch fest durch den Hebelantrieb kontrolliert und gesteuert.

Vorzugsweise werden die beiden Hebel unter 180° versetzt an derselben Antriebswelle exzentrisch angelenkt. Wenn beide Hebel gleichgeformte Gleitflächen aufweisen und in entsprechenden Führungsflächen gleiten, vollführen sie dementsprechend identische Vorwärt- und Rückwärtsbewegungen, die um eine halbe Zyklus länge zeitlich zueinander versetzt durchgeführt werden.

In einer bevorzugten Ausführungsform wird die Antriebswelle für den exzentrischen Antrieb des Hebels mit einem Schneckenrad ausgestattet, in das eine Schnecke eingreift. Hierdurch sind zum einen extreme Übersetzungen möglich, was insbesondere bei der Verwendung in einem erfindungsgemäßen Duftverdunster, wo ein Motor für den Ventilator und die Rotation des Plattenstapels eingesetzt wird, notwendig ist. Zum anderen stellt ein Schneckenradantrieb ein sogenanntes selbsthemmendes Getriebe dar, wodurch in Verbindung mit dem Zwei-Hebelsystem, bei dem sich ständig ein Hebel mit dem Zahnrad des rotierenden Gegenstandes in Eingriff befindet, eine Verriegelung des rotierenden Gegenstandes, beispielsweise eines Plattenstapels, bei ausgeschaltetem Antrieb ergibt.

Vorzugsweise wird die Schnecke über eine elastische Kupplung mit der Antriebswelle eines Motors verbunden. Hierdurch braucht die Antriebswelle nicht vollständig exakt in Flucht mit der Welle der Schnecke zu stehen. Größere Toleranzen bei der Fertigung eines erfindungsgemäßen Antriebs sind hierbei möglich, was unter anderem eine Kostenersparnis mit sich bringt.

Ein Ausführungsbeispiel der Erfindung ist in der Zeichnung dargestellt und wird anhand der nachfolgenden Beschreibung näher erläutert.

Im einzelnen zeigen
- Fig. 1: einen schematischen Querschnitt durch einen erfindungsgemäßen Duftverdunster;
- Fig. 2: einen Querschnitt gemäß Fig. 1 während der Herausnahme einer Wechselkassette;
- Fig. 3: einen schematischen Querschnitt durch eine Wechselkassette mit aufgesetzter Abschlußhaube;
- Figuren 4 und 5: einen schematischen Querschnitt durch ein Antriebssystem in verschiedenen Hebelpositionen;
- Fig. 6: einen schematischen vertikalen Querschnitt durch einen Teil der Antriebsvorrichtung und
- Fig. 7: eine schematische Darstellung eines Duftverdunsters mit zwei Plattenstapel und doppeltem Antrieb.

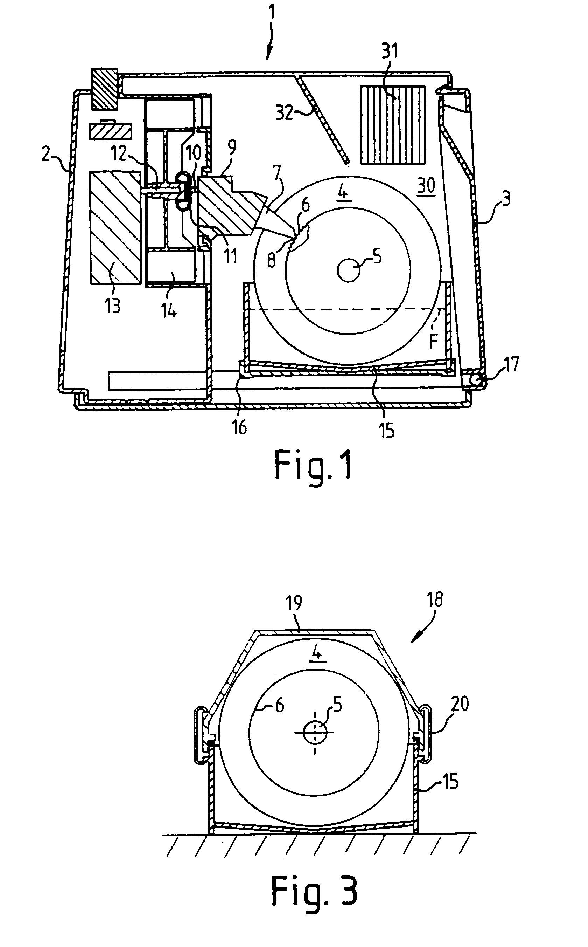

Der Duftverdunster 1 gemäß Fig. 1 weist ein Gehäuse 2 mit Öffnungsklappe 3 auf. Im Inneren des Gehäuses 2 ist ein Plattenstapel 4 aus lamellenförmig hintereinander angeordneten kreisförmigen Scheiben, dargestellt. In der gezeigten Darstellung ist hierbei nur die vorderste Platte sichtbar. Mit der Achse 5 des Plattenstapels 4 ist eine Zahnscheibe oder Zahnrad 6 fest verbunden. In das Zahnrad 6 greift ein Hebel 7 mit seiner Hebelspitze 8 ein. Das hintere Ende des Hebels 7 ist in einem Getriebegehäuse 9 verborgen. Das Getriebegehäuse 9 wird von einer Getriebeantriebswelle 10 durchsetzt, die über eine elastische Kupplung 11 mit einer Motorantriebswelle 12 eines Elektromotors 13 verbunden ist. Auf der Motorantriebswelle 12 ist ein Flügelrad 14 als Ventilator aufgesetzt.

Der Plattenstapel 4 ist mit seiner Achse 5 in den nicht dargestellten Stirnseiten einer Duftstoffwanne 15 drehbar gelagert. Die Duftstoffwanne 15 steht in einer verschiebbaren Schubladenhalterung 16, an der, wie nicht näher dargestellt, auch die Öffnungsklappe 3 an ihrer Drehachse 17 schwenkbar befestigt ist.

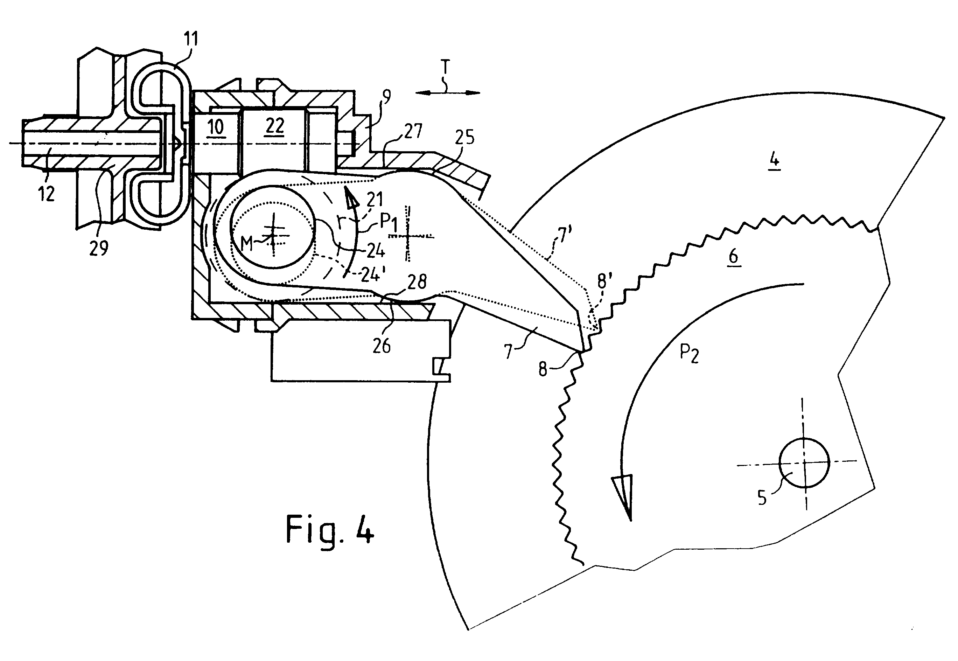

In Fig. 3 ist eine komplette Wechselkassette 18 für das Duftstoffbad dargestellt. Sie umfaßt die Duftstoffwanne 15 mit dem darin gelagerten Plattenstapel 4 sowie eine Kassettenhaube 19, die mittels Verschlußklammern 20 auf der Duftstoffwanne 15 befestigt ist.

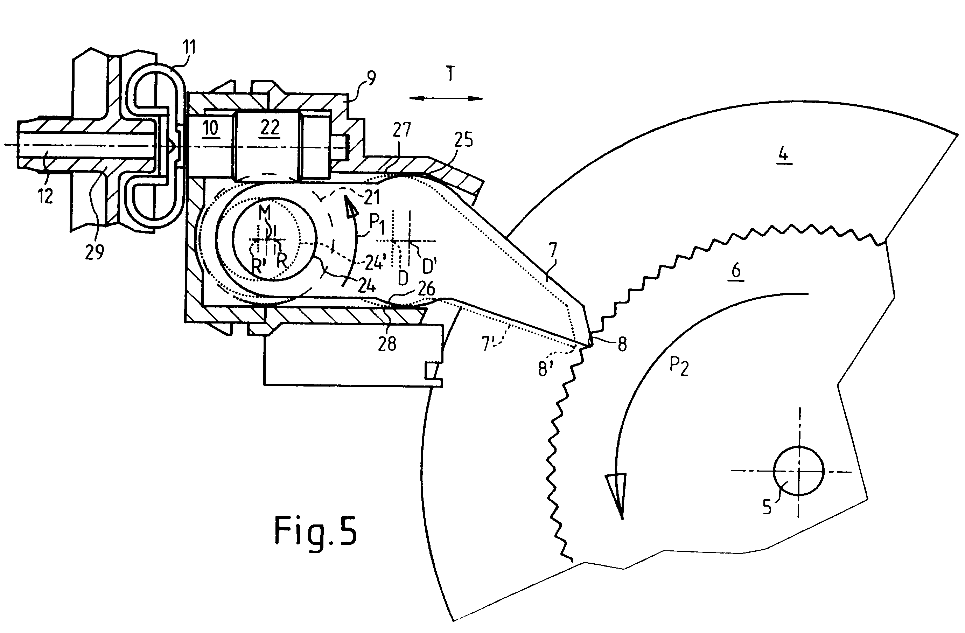

Die Figuren 4 und 5 zeigen einen Hebelantrieb in vergrößerter Darstellung. Ein vorderer Hebel 7 ist hierbei mit durchgezogenen Linien dargestellt, während ein zweiter hinter dem Hebel 7 befindlicher Hebel 7' mit punktierten Linien gekennzeichnet ist.

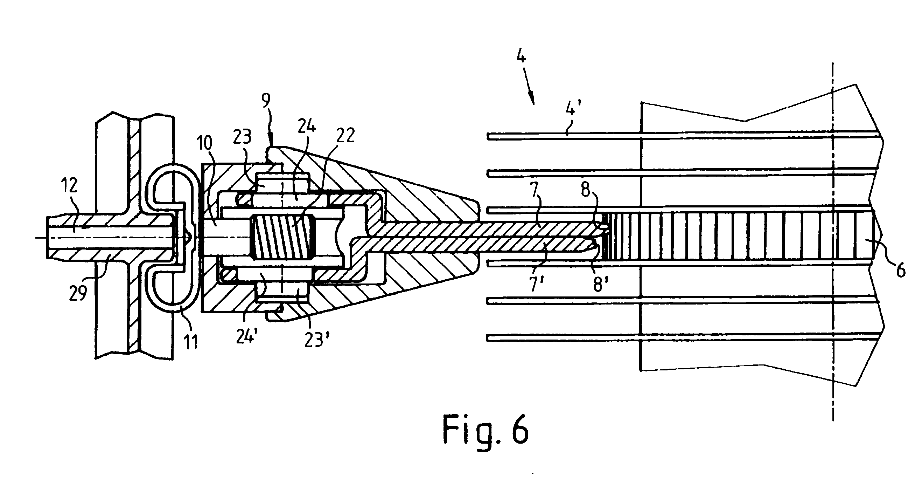

Ein Schneckenrad 21, das eine Schnecke 22 kämmt, ist mit gestrichelten Linien gezeichnet. Das Schneckenrad 21 ist drehfest mit einer in den Figuren 4 und 5 nicht näher dargestellten, jedoch in Fig. 6 erkennbaren Exzenterantriebswelle 23 drehfest verbunden oder in diese eingearbeitet. Auf der Exzenterantriebswelle 23 sitzen zwei als Ringschultern ausgebildete Exzenter 24, 24', deren Mittelachsen R, R' gegenüber der Mittelachse M der Exzenterantriebswelle 23 versetzt sind, so daß sich eine Exzentrizität ergibt. Die Exzenter 24, 24' durchsetzen zwei entsprechende Bohrungen in dem jeweiligen Hebel 7, 7'. Jeder Hebel 7 bzw. 7' ist jeweils mit einer oberen 25 und unteren 26 Gleitfläche versehen. Diese liegen auf oberen 27 und unteren 28 Führungsflächen auf, die in das Getriebegehäuse 9 eingearbeitet sind.

Fig. 6 zeigt die drehfest mit der Getriebeantriebswelle 10 verbundene oder in diese eingearbeitete Schnecke 22. Die elastische Kupplung 11 ist über eine Steckhülse 29 des Flügelrads 14 mit der Motorantriebswelle 12 verbunden. Außerdem ist hier die lamellenartige Anordnung der einzelnen Scheiben 4' des Plattenstapels 4 ersichtlich.

Im Betrieb des erfindungsgemäßen Duftverdunsters 1 befindet sich in der Duftstoffwanne 15, wie mit der Linie F als Flüssigkeitspegel dargestellt ist, ein flüssiger Duftstoff. Innerhalb des Duftstoffes liegt teilweise ein Plattenstapel 4. Dieser ist über seine Achse 5 drehbar in der Duftstoffwanne 15 gelagert. Er wird über die Hebel 7, 7' und das Zahnrad 6 in eine Drehbewegung versetzt. Hierbei kommt die mit Duftstoff benetzte Oberfläche des Plattenstapels 4 mit der Luft im Verdunstungsraum 30 in Berührung, so daß der Duftstoff von der Oberfläche in die Luft des Verdunstungsraums 30 verdunstet.

Durch das Flügelrad 14 wird über eine nicht näher dargestellte Öffnung im Gehäuse 2 des Duftverdunsters 1 Luft angesaugt und über den Plattenstapel 4 geblasen. Hierdurch kommt es zu einer verstärkten Verdunstung von Duftstoff. Die mit Duftstoff angereicherte Luft im Verdunstungsraum 30 entweicht anschließend durch entsprechende Lüftungsöffnungen, wie sie durch die Lüftungsschlitze 31 schematisch angedeutet sind. Eine Blende 32 lenkt hierbei den Luftstrom zusätzlich in Richtung auf den Plattenstapel ab.

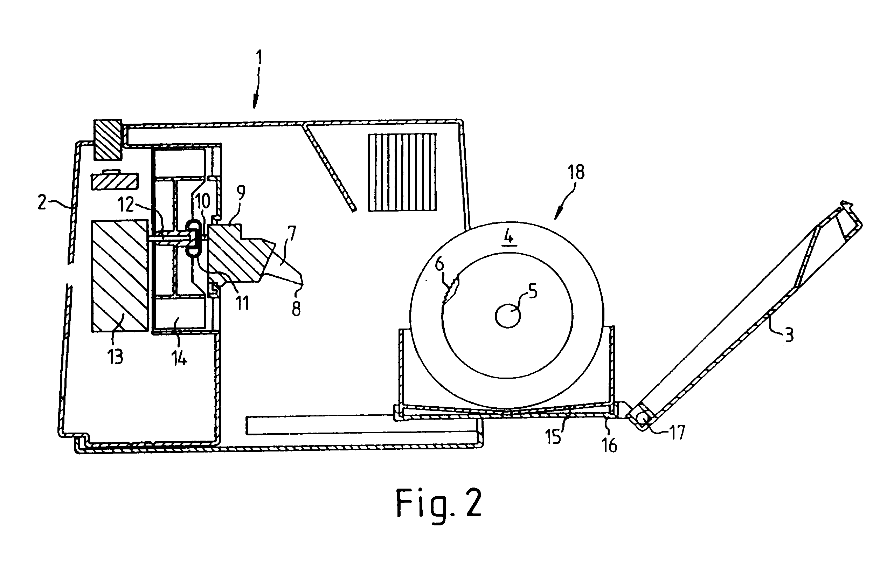

Falls der Duftstoff gewechselt werden soll, so kann die Klappe 3, wie in Fig. 2 dargestellt, nach unten heruntergeklappt werden und die Duftstoffwanne 15 auf der Schubladenhalterung 16 herausgezogen werden. Der Plattenstapel 4 bleibt hierbei in der Duftstoffwanne 15 gelagert. Anschließend kann die Wechselkassette 18, die die Duftstoffwanne 15 mitsamt ihres Plattenstapels 4 umfaßt, aus der Schubladenhalterung 16 entnommen werden und mit einer Kassettenhaube 19 verschlossen werden. Diese Wechselkassette 18 ist somit bereit für eine längere Lagerung oder für einen Transport. Eine andere baugleiche Wechselkassette oder auch dieselbe Wechselkassette 18 kann anschließend wieder in den Duftverdunster 1 in umgekehrter Reihenfolge eingesetzt werden.

Der Antrieb für den Plattenstapel 4 geschieht über das Zahnrad 6 und die Hebel 7, 7'. Die Hebel 7, 7' werden mit Hilfe des Elektromotors 13, der auch das Flügelrad 14 treibt, angetrieben. Dies geschieht über die Steckhülse 29 des Flügelrads 14 und die elastische Kupplung 11, wodurch die Getriebeantriebswelle 10 angetrieben wird.

Hierdurch dreht sich die Schnecke 22 und somit auch die Exzenterantriebswelle 23 in Richtung des Pfeils P₁. Die Exzenter 24 vollführen dabei eine kreisförmige Bewegung. Durch diese kreisförmige Bewegung werden die Hebel 7, 7' sowohl in eine Kippbewegung um den Drehpunkt D, D' als auch in eine Translationsbewegung in Richtung des Doppelpfeils T versetzt. Während dieser Bewegungen wird jeder Hebel durch die obere und untere Führungsfläche 26, 28 geführt, auf denen er mit seinen oberen bzw. unteren Gleitflächen 25, 26 aufliegt. Durch die besondere Formgebung der Führungsflächen 27, 28 sowie der Gleitflächen 25, 26 in Verbindung mit der Auslenkung der Mittelachsen R, R' der Exzenter 24, 24' von der Mittelachse M der Drehachse 23 ist nahezu jede beliebige Bahnkurve der Hebelspitzen 8, 8' möglich. Von großem Vorteil ist hierbei eine elliptische Bahnkurve, so daß die Hebelspitzen 8, 8' in der Vorwärtsbewegung, d. h. in der vorliegenden Darstellung in der Bewegung nach unten, sich in Eingriff mit dem Zahnrad 6 befinden. Bei der Rückwärtsbewegung, d. h. vorliegend bei der Bewegung nach oben, befinden sich die Hebelspitzen 8, 8' sodann außer Eingriff mit dem Zahnrad 6 und berühren dessen Zähne nicht.

Die Darstellungen der Figuren 4 + 5 zeigen die Hebel 7, 7' in verschiedenen Stellungen während des Antriebsvorgangs, bei dem der Plattenstapel 4 in Richtung des Pfeils P₂ gedreht wird. Da die Auslenkung der Exzenter 24, 24' gegenüber der Mittelachse M der Exzenterantriebswelle 23 um 180° zueinander versetzt liegt, d. h. die Mittelachsen R, R' der Exzenter 24, 24' und die Mittelachse M der Exzenterantriebswelle 23 liegen auf einer Geraden, vollzieht sich die Bewegung des Hebels 7' um einen halben Bewegungszyklus versetzt zur Bewegung des Hebels 7. Hierdurch befindet sich jeweils mindestens eine Hebelspitze 8 in Eingriff des Zahnrads 6, so daß dieses nicht nur in Richtung P₂ mitgenommen, sondern auch in jeder Stellung des Antriebs gegenüber anderen Bewegungen, beispielsweise einem Zurückdrehen entgegen dem Drehsinn von P₂ verriegelt ist.

Die Hebelspitzen 8, 8' sind nach unten abwärts gerichtet, so daß Flüssigkeit, die durch das Eintauchen des Zahnrads 6 in den Duftstoff innerhalb der Duftstoffwanne 15 eventuell zu den Hebelspitzen 8, 8' gefördert wird, wieder hinunter auf das Zahnrad 6 abläuft. Die einzigen Stellen, die also somit mit Duftstoff in Verbindung kommen können, sind lediglich die Spitzen der Hebel 7, 7'. Es besteht somit keine Gefahr, daß die restlichen Komponenten des Antriebs in irgendeiner Phase des Betriebs des Duftverdunsters mit flüssigem Duftstoff in Berührung kommen.

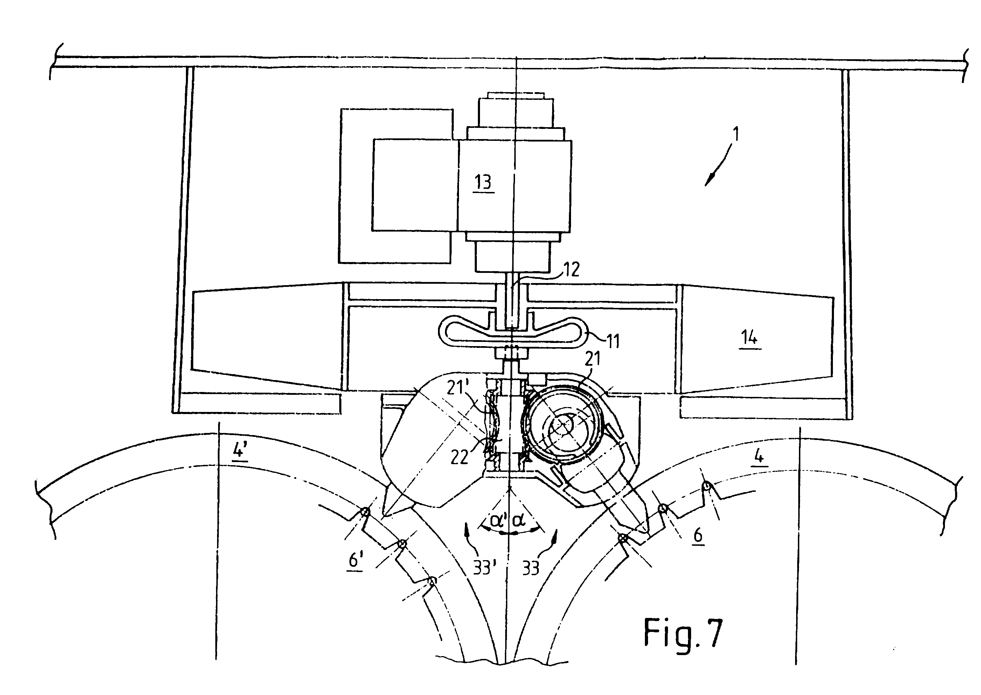

Der dargestellte Antrieb für den Plattenstapel 4 läßt sich auch außerhalb eines erfindungsgemäßen Duftverdunsters einsetzen. Das dargestellte System läßt sich ohne weiteres, auch in der nachfolgend beschriebenen Ausführung gem. Fig. 7, auf jeden rotierenden Gegenstand anwenden, bei dem ein erfindungsgemäßes Antriebssystem Vorteile bietet. Dies kann insbesondere bei chemischen Produktionsanlagen der Fall sein, wo ebenfalls häufig Walzen, Wellen oder dergleichen innerhalb eines Flüssigkeitsbades drehen müssen, und somit ebenfalls das Problem auftritt, daß der Antrieb nicht mit der ggf. aggressiven Flüssigkeit nicht oder nur punktuell in Berührung kommen darf.

In Fig. 7 ist eine weitere Ausführungsform eines erfindungsgemäßen Duftverdunsters dargestellt. Hierbei stehen zwei Plattenstapel 4, 4' mit jeweils einem Zahnrad 6, 6' parallel zueinander. Sie werden über einen doppelten Hebelantrieb 33, 33' angetrieben. Die Hebelantriebe 33 entsprechen den bisherigen oben beschriebenen einfachen Hebelantrieben. Sie werden über eine gemeinsame Schnecke 22 und die beiden Schneckenräder 21, 21' angetrieben. Im vorliegenden Fall ist die Achse der Schnecke 22 und damit auch der Getriebeantriebswelle 10 senkrecht angeordnet, wobei die beiden Hebelantriebe 33, 33' unter einem Winkel a bzw. a' schräg zur senkrechten Achse der Getriebeantriebswelle stehen. Entsprechend ist auch das Flügelrad 14 sowie der Elektromotor mit senkrecht verlaufender Achse angeordnet.

Durch die Verwendung zweier paralleler Plattenstapel wird die benetzte Oberfläche insgesamt vergrößert, wobei nach wie vor nur ein Antriebsmotor und ein Ventilator notwendig ist. Durch die genannte räumliche Anordnung der Plattenstapel ist eine kompakte Bauweise bei hoher Effizienz eines erfindungsgemäßen Duftverdunsters möglich.
- 1: Duftverdunster
- 2: Gehäuse
- 3: Öffnungsklappe
- 4: Plattenstapel
- 5: Achse
- 6: Zahnrad
- 7: Hebel
- 8: Hebelspitze
- 9: Getriebegehäuse
- 10: Getriebeantriebswelle
- 11: elastische Kupplung
- 12: Motorantriebswelle
- 13: Elektromotor
- 14: Flügelrad
- 15: Duftstoffwanne
- 16: Schubladenhalterung
- 17: Drehachse
- 18: Wechselkassette
- 19: Kassettenhaube
- 20: Verschlußklammer
- 21: Schneckenrad
- 22: Schnecke
- 23: Exzenterantriebswelle
- 24: Exzenter
- 25: obere Gleitfläche
- 26: untere Gleitfläche
- 27: obere Führungsfläche
- 28: untere Führungsfläche
- 29: Steckhülse
- 30: Verdunstungsraum
- 31: Lüftungsschlitze
- 32: Blende
- 33: Hebelantrieb

## Patentansprüche

1. Duftverdunster insbesondere für Toiletten, mit einem innerhalb der Raumluft zu verteilenden Flüssigduftstoff, wobei ein rotierender Plattenstapel (4) als benetzbare feste Oberfläche vorgesehen ist, der in ein mit flüssigem Duftstoff versehenes Duftstoffreservoir (15) teilweise eintaucht und Umgebungsluft an die benetzte Oberfläche mittels eines Ventilators vorbeigeführt wird, dadurch gekennzeichnet, daß das Duftstoffreservoir als separate, dem Gehäuse (2) des Duftverdunsters (1) entnehmbare Duftstoffwanne (15) ausgebildet ist, in welches der Plattenstapel eingesetzt ist und daß der Antrieb für den Plattenstapel über einen Hebelantrieb (7, 6) erfolgt.

2. Duftverdunster nach Anspruch 1, dadurch gekennzeichnet, daß ein Flügelrad (14) als Ventilator zur Belüftung des Verdunstungsraumes (30) vorgesehen ist.

3. Duftverdunster nach Anspruch 1 oder 2, dadurch gekennzeichnet, daß für den Antrieb des Plattenstapels (4) und den Antrieb des Flügelrads (14) derselbe Motor (13) vorgesehen ist.

4. Duftverdunster nach einem der vorhergehenden Ansprüche dadurch gekennzeichnet, daß eine Steuerung vorgesehen ist, die einen intermittierenden Betrieb erlaubt.

5. Duftverdunster nach einem der vorhergehenden Ansprüche, dadurch gekennzeichnet, daß eine Steuerung vorgesehen ist, bei der die Betriebszeit des Duftverdunsters durch Sensoren und/oder durch ein oder mehrere Zeitintervalle steuerbar ist.

6. Duftverdunster nach einem der vorhergehenden Ansprüche, dadurch gekennzeichnet, daß wenigstens eine Wechselkassette (18) für das Duftstoffbad vorgesehen ist.

7. Duftverdunster nach einem der vorhergehenden Ansprüche, dadurch gekennzeichnet, daß zwei oder mehrere Plattenstapel vorgesehen sind.

8. Duftverdunster nach einem der vorhergehenden Ansprüche, dadurch gekennzeichnet, daß ein Antrieb für den rotierenden Plattenstapel vorgesehen ist, der wenigstens einen schwenkbar gelagerten Hebel (7) und ein Zahnrad (6) umfaßt, wobei das Zahnrad (6) drehfest mit dem rotierenden Plattenstapel (4) verbunden ist und eine Vorrichtung zur Lagerung und Führung des Hebels (24 bis 28) für eine in das Zahnrad eingreifende und mitnehmende Vorwärtsbewegung des Hebels (7) vorhanden ist.

9. Duftverdunster nach Anspruch 8, dadurch gekennzeichnet, daß der Hebel (7) mit seiner zum Zahnrad (6) hinweisenden Spitze (8) in das Zahnrad (6) eingreift und diese Spitze (8) des Hebels (7) in jeder Stellung des Hebels (7) nach unten ausgerichtet ist.

10. Duftverdunster nach einem der Ansprüche 8 oder 9, dadurch gekennzeichnet, daß die Vorrichtung zur Lagerung und Führung (24 bis 28) des Hebels für eine zwischen zwei Vorwärtsbewegungen des Hebels stattfindende Rückwärtsbewegung eingerichtet ist, bei der sich der Hebel (7) nicht mehr in Eingriff mit dem Zahnrad (6) befindet und dieses während der Rückwärtsbewegung nicht berührt.

11. Duftverdunster nach einem der Ansprüche 8 bis 10, dadurch gekennzeichnet, daß die Vorrichtung zur Lagerung und Führung des Hebels (7) Gleitflächen (25, 26) am Hebel (7) und eine Halterung (9) mit Führungsflächen (27, 28) umfaßt.

12. Duftverdunster nach einem der Ansprüche 8 bis 11, dadurch gekennzeichnet, daß der Hebel an einem Ende mit einer Antriebswelle (23) über einen Exzenter (24) gelenkig verbunden ist.

13. Duftverdunster nach einem der Ansprüche 8 bis 12, dadurch gekennzeichnet, daß zwei Hebel (7, 7') vorhanden sind.

14. Duftverdunster nach einem der Ansprüche 8 bis 13, dadurch gekennzeichnet, daß die Antriebswelle (23) für den Hebel (7) mit einem Schneckenrad (21) versehen ist, in das eine Schnecke (22) eingreift.

15. Duftverdunster nach einem der Ansprüche 8 bis 14, dadurch gekennzeichnet, daß die Schnecke (22) über eine elastische Kupplung (11) mit der Antriebswelle (12) eines Motors verbunden ist.

## Claims

1. Scent vaporizer, in particular for toilets, with a liquid scent which is to be distributed within the surrounding air, wherein a rotating stack of plates (4) is provided as a wettable solid surface which is partially immersed in a scent reservoir (15) provided with liquid scent and ambient air is guided past the wetted surface by means of a fan, characterized in that the scent reservoir is designed as a separate scent trough (15) which can be removed from the casing (2) of the scent vaporizer (1) and in which the stack of plates is inserted, and in that the stack of plates is driven via a lever drive (7, 6).

2. Scent vaporizer according to claim 1, characterized in that an impeller wheel (14) is provided as a fan for aerating the evaporation chamber (30).

3. Scent vaporizer according to claim 1 or 2, characterized in that the same motor (13) is provided for driving the stack of plates (4) and driving the impeller wheel (14).

4. Scent vaporizer according to one of the preceding claims, characterized in that a controller which allows intermittent operation is provided.

5. Scent vaporizer according to one of the preceding claims, characterized in that a controller is provided in which the operating time of the scent vaporizer can be controlled by sensors and/or by one or more time intervals.

6. Scent vaporizer according to one of the preceding claims, characterized in that at least one cartridge (18) is provided for the scent bath.

7. Scent vaporizer according to one of the preceding claims, characterized in that two or more stacks of plates are provided.

8. Scent vaporizer according to one of the preceding claims, characterized in that the rotating stack of plates is provided with a drive comprising at least one pivotally mounted lever (7) and one gear-wheel (6), the gear-wheel (6) being rotationally engaged with the rotating stack of plates (4) and a device being provided for supporting and guiding the lever (24 to 28) for a forward movement of the lever (7) which engages in and entrains the gear-wheel.

9. Scent vaporizer according to claim 8, characterized in that the lever (7) with its tip (8) directed toward the gear-wheel (6) engages in the gear-wheel (6) and this tip (8) of the lever (7) is orientated downwardly in each position of the lever (7).

10. Scent vaporizer according to one of claims 8 or 9, characterized in that the device for supporting and guiding (24 to 28) the lever is adjusted for a backward movement taking place between two forward movements of the lever, during which the lever (7) no longer engages with the gear-wheel (6) and does not touch it during the backwards movement.

11. Scent vaporizer according to one of claims 8 to 10, characterized in that the device for supporting and guiding the lever (7) comprises sliding faces (25, 26) on the lever (7) and a mounting (9) with guide faces (27, 28).

12. Scent vaporizer according to one of claims 8 to 11, characterized in that the lever is articulated at one end to a drive shaft (23) via a cam (24).

13. Scent vaporizer according to one of claims 8 to 12, characterized in that two levers (7, 7') are provided.

14. Scent vaporizer according to one of claims 8 to 13, characterized in that the drive shaft (23) for the lever (7) is provided with a worm wheel (21) in which a worm (22) engages.

15. Scent vaporizer according to one of claims 8 to 14, characterized in that the worm (22) is connected via a flexible coupling (11) to the drive shaft (12) of a motor.

## Revendications

1. Evaporateur de parfum, en particulier pour des toilettes, ayant un parfum liquide à répartir dans l'air ambiant, une pile de plaques rotative (4) étant prévue comme surface solide mouillable, qui plonge partiellement dans un réservoir (15) contenant du parfum liquide, et l'air ambiant étant amené à la surface mouillée au moyen d'un ventilateur,
caractérisé en ce que le réservoir de parfum est réalisé comme cuvette de parfum séparée (15), pouvant être retirée du boîtier (2) de l'évaporateur de parfum (1), dans lequel la pile de plaques est montée, et en ce que l'entraînement pour la pile de plaques a lieu par l'intermédiaire d'un entraînement à levier (7,6).

2. Evaporateur de parfum selon la revendication 1,
caractérisé en ce qu'un rotor (14) est prévu comme ventilateur pour l'aération de l'espace d'évaporation (30).

3. Evaporateur de parfum selon la revendication 1 ou 2,
caractérisé en ce que le même moteur (13) est prévu pour l'entraînement de la pile de plaques (4) et l'entraînement du rotor (14).

4. Evaporateur de parfum selon une des revendications précédentes,
caractérisé en ce qu'il est prévu une commande qui permet un fonctionnement intermittent.

5. Evaporateur de parfum selon une des revendications précédentes,
caractérisé en ce qu'il est prévu une commande pour laquelle le temps de fonctionnement de l'évaporateur de parfum peut être commandé par des capteurs et/ou par un ou plusieurs intervalles de temps.

6. Evaporateur de parfum selon une des revendications précédentes,
caractérisé en ce qu'au moins une cassette interchangeable (18) est prévue pour le parfum.

7. Evaporateur de parfum selon une des revendications précédentes,
caractérisé en ce que deux ou plusieurs piles de plaques sont prévues.

8. Evaporateur de parfum selon une des revendications précédentes,
caractérisé en ce qu'il est prévu un entraînement pour la pile de plaques rotative, qui comporte au moins un levier (7) monté de façon pivotante et une roue dentée (6), la roue dentée (6) étant reliée, de façon solidaire en rotation, à la pile de plaques rotative (4), et un dispositif pour monter et guider le levier (24 à 28) étant prévu pour un mouvement d'avance du levier (7) engageant et entraînant la roue dentée.

9. Evaporateur de parfum selon la revendication 8,
caractérisé en ce que le levier (7) s'engage dans la roue dentée (6) par son extrémité (8) tournée vers la roue dentée (6) et cette extrémité (8) du levier (7), dans chaque position du levier (7), est orientée vers le bas.

10. Evaporateur de parfum selon une des revendications 8 ou 9,
caractérisé en ce que le dispositif pour monter et guider (24 à 28) le levier est prévu pour un mouvement en arrière ayant lieu entre deux mouvements d'avance du levier, pour lequel le levier (7) ne se trouve plus en engagement avec la roue dentée (6) et n'est pas en contact avec celle-ci pendant le mouvement en arrière.

11. Evaporateur de parfum selon une des revendications 8 à 10,
caractérisé en ce que le dispositif pour monter et guider le levier (7) comporte des surfaces de glissement (25,26) sur le levier (7) et un support (9) ayant des surfaces de guidage (27,28).

12. Evaporateur de parfum selon une des revendications 8 à 11,
caractérisé en ce que le levier est relié de façon articulée, à une extrémité, à un arbre d'entraînement (23) par l'intermédiaire d'un excentrique (24).

13. Evaporateur de parfum selon une des revendications 8 à 12,
caractérisé en ce que deux leviers (7,7') sont prévus.

14. Evaporateur de parfum selon une des revendications 8 à 13,
caractérisé en ce que l'arbre d'entraînement (23) pour le levier (7) est muni d'une roue à denture hélicoïdale (21) dans laquelle s'engage une vis sans fin (22).

15. Evaporateur de parfum selon une des revendications 8 à 14,
caractérisé en ce que la vis sans fin (22) est reliée, par l'intermédiaire d'un accouplement élastique (11), à l'arbre d'entraînement (12) d'un moteur.
